# EUROPÄISCHE PATENTSCHRIFT

(11) **EP 0 728 458 B1**
(45) Veröffentlichungstag und Bekanntmachung des Hinweises auf die Patenterteilung: **10.10.2001**
(21) Anmeldenummer: 96102363.7
(22) Anmeldetag: 16.02.1996
(51) Int. Cl.: A61K 6/093

(54) **Stoffgemisch für Kofferdamwerkstoff, Kofferdamwerkstoff, dessen Verwendung und Herstellung**
Composition and material for protective dams, their use and manufacture
Compositions et matériau pour digues de caoutchouc, leur utilisation et leur fabrication

(30) Priorität: 17.02.1995 DE 19505496
(43) Veröffentlichungstag der Anmeldung: 28.08.1996
(73) Patentinhaber: ROEKO GmbH + Co. Dentalerzeugnisse, 89122 Langenau (DE)
(72) Erfinder: Mannschedel, Werner, D-89129 Langenau (DE)
(74) Vertreter: Boeters, Hans Dietrich, Dr.

(56) Entgegenhaltungen:
- EP-A- 0 522 341
- WO-A-91/00723
- WO-A-94/13222
- DE-A- 2 316 589
- DE-A- 3 902 817
- US-A- 3 310 521

## Beschreibung

Die Erfindung betrifft ein Stoffgemisch für einen Kofferdam-Werkstoff, den Werkstoff, dessen Verwendung und Herstellung.

Bei Kofferdam handelt es sich um ein in der Zahnheilkunde übliches Hilfsmittel, beispielsweise für zahnärztliche Operationen. Synonyme Bezeichnungen sind Rubberdam, Spanngummi, Gummilappen, Gummiplatte und Gummituch. Der Kofferdam wird bei einer Dentalbehandlung in der Mundhöhle des Patienten im Bereich des zu behandelnden Zahnes oder einer zu behandelnden Zahngruppe angeordnet, wobei er unterschiedlichste Funktionen übernehmen kann. Beispielsweise soll er den Patienten vor einer unbeabsichtigten Aspiration oder einem unbeabsichtigten Verschlucken schützen, ferner einen Infektionsschutz für Patient, behandelndes Personal und Zahnarzt gewähren, zu einem aseptischen Arbeitsfeld und zur Trockenlegung des Eingriffbereichs, zur Retraktion und zum Schutz von Weichgewebe und zur Verbesserung der Sicht und einem erweiterten Zugang beitragen; verwiesen sei beispielsweise auf Winkler, Kofferdam in Theorie und Praxis, Quintessenz-Verlags GmbH, 1991 beispielsweise Seite 17 Abb. 7; sowie Denis & Ott in Dtsch. Zahnärztl. Z., (1993) 306-308.

Ein derartiger Kofferdam kann zusätzlich mit einer Halteeinrichtung vorgesehen werden, um ihn im Zahnbereich als quadratisches, rechteckiges oder rundes Schutzelement zu befestigen.

Hauptbestandteil des Kofferdams ist üblicherweise Kautschuk, der aus Latexsaft gewonnen wird. Zur Herstellung wird das flüssige Rohlatex zu einem dünnen Film koaguliert, der einer Hitzevulkanisation unterworfen wird. Das auf diese Weise hergestellte dünne Gummimaterial kommt in Form von Platten oder Rollen in den Handel.

Ein großes Problem bei der Verwendung von üblichem Kofferdam, der unter Verwendung von Kautschuk gewonnen ist, sind allergische Reaktionen. Es ist anzunehmen, daß Allergien auf die komplexe Zusammensetzung des Latexsaftes und auf Zusätze zurückzuführen sind, die bei der Vulkanisation des Latexsaftes verwendet werden.

Aus DE-A-3 423 823 und EP-A-0 268 347 ist bereits die Verwendung von vernetzbaren Siliconen mit einem Gehalt an Kieselsäure als Füllstoff und Silicon-Verarbeitungshilfsmitteln als dentale Abformmassen bekannt. Nach der DE-A-3 423 823 kann man eine Basispaste und eine Katalysatorpaste vorsehen, die Platin als Katalysator enthält. Die beiden Pasten werden beim Einsatz miteinander vermischt und härten bei Raumtemperatur aus (Beispiele 3 und 5). Die mit EP-A-0 268 347 gegebene Lehre entspricht diesem Stand der Technik und arbeitet mit Platin-Schwarz als Katalysator.

Ferner ist es aus US-A-5 098 299 bereits bekannt, Rubberdam mit Hilfe einer Zusammensetzung abzudichten, auszubessern oder anzupassen, die ein Material mit einem Gehalt an beispielsweise Polydimethylsiloxan, Füllmittel, wie Glasteilchen, einem hydrophilisierenden niedermolekularen aliphatischen Glykol sowie einem Zellulosematerial für die Homogenität darstellt. Die Plastizität dieses Materials gestattet es, in den Mund eingebrachte Massen in situ anzupassen und anzuformen (Spalte 12 unten bis Spalte 13 oben), wozu man lichthärtende Materialien wählt (Spalte 10 Zeile 33). Die dieser bekannten Zusammensetzung abverlangten Eigenschaften sollen beispielsweiese für 0,2 bis 4 h garantiert werden (Example 1).

Eine Aufgabe der Erfindung ist es nun, ein Stoffgemisch für einen Werkstoff für Kofferdam sowie einen Kofferdam-Werkstoff vorzusehen. Diese Gegenstände sollen einfach herstellbar und einfach verarbeitbar sein und möglichst keine unerwünschten Körperreaktionen auslösen, insbesondere keine Allergien.

Ein weiteres Ziel der Erfindung besteht darin, ein produktionstechnisch einfaches Verfahren zur Herstellung des genannten Stoffgemischs und Werkstoffs vorzusehen.

Gemäß einer Ausführungsform wird die der Erfindung zugrundeliegende Aufgabe durch ein Stoffgemisch für einen Werkstoff für Kofferdam gelöst, das gekennzeichnet ist durch
(a) 56 bis 96 Gew.-% Silikonkautschuk (Polymer),
(b) 44 bis 4 Gew.-% Kieselsäure (Füllstoff),
(c) 1 bis 7 Gew.-% übliches Silikonkautschuk-Verarbeitungshilfsmittel (bezogen auf (a) und (b) als 100 %) und
(d) 0,1 bis 1,1 Gew.-% eines Radikale bildenden Vernetzers (bezogen auf (a) und (b) als 100 %), wobei der Vernetzer im Gemisch mit den Komponenten (a) bis (c) oder getrennt davon vorliegen kann.

Gemäß einer speziellen Auführungsform kann das Stoffgemisch 8 bis 28 und insbesondere etwa 20 Gew.-% Kieselsäure enthalten. Die Kieselsäure dient insbesondere dazu, Elastizität, Rückstellvermögen und Reißfestigkeit des Werkstoffs einzustellen. Besonders bevorzugt ist hochaktive pyrogene Kieselsäure.

Besonders bevorzugt ist ein Stoffgemisch für einen transparenten Werkstoff. Ein Gehalt an Farbpigmenten ist möglich. Bei einem transparenten Werkstoff liegt die obere Grenze jedoch dort, wo die Transparenz beeinträchtigt würde. Es ist selbstverständlich, daß Transparenz die Beobachtung des Operationsumfeldes erleichtert.

Das erfindungsgemäße Stoffgemisch kann ein Öl, insbesondere ein Silikonöl, als Silikonkautschuk-Verarbeitungshilfsmittel enthalten. Als Vernetzer kann man Peroxide einsetzen, beispielsweise einen in der Gummiindustrie üblichen Vernetzer.

Eine beispielhafte Rezeptur eines erfindungsgemäßen Stoffgemischs sieht
(a) etwa 80 Gew.-% Silikonkautschuk,
(b) etwa 20 Gew.-% Kieselsäure,
(c) etwa 4 Gew.-% Silikonkautschuk-Verarbeitungshilfsmittel (auf Basis von (a) und (b) als 100 %) und
(d) etwa 0,6 Gew.-% eines Radikale bildenden Vernetzers (auf Basis von (a) und (b) als 100 %) vor.

Die der Erfindung zugrundeliegende Aufgabe wird gemäß einer weiteren Ausführungsform durch einen Kofferdam-Werkstoff gelöst, der durch Heißvulkanisieren aus einem erfindungsgemäßen Stoffgemisch erhalten werden kann. Sofern man für den Kofferdam-Werkstoff ein erfindungsgemäßes Stoffgemisch mit den Merkmalen (a) bis (c) heranzieht, kann man für dieses Heißvulkanisieren außer Radikale bildenden Vernetzern auch Metalle als Vernetzer verwenden, beispielsweise Platin.

Der erfindungsgemäße Werkstoff ist vorzugsweise transparent. Er kann als Flachmaterial vorgesehen werden, insbesondere in Form runder, rechteckiger oder quadratischer Platten, und insbesondere mit den Ausmaßen von etwa 150 x etwa 150 mm, wobei die Stärke des erfindungsgemäßen Werkstoffs 0,1 bis 0,8 mm betragen kann.

Zur Herstellung eines erfindungsgemäßen Stoffgemischs und eines erfindungsgemäßen Kofferdam-Werkstoffs geht man so vor, daß man
(i)
   (a) 56 bis 96 Gew.-% Silikonkautschuk (Polymer),
   (b) 44 bis 4 Gew.-% Kieselsäure (Füllstoff) und 1 bis 7 Gew.-% üblicher Silikonkautschuk-Verarbeitungshilfsmittel (bezogen auf (a) und (b) als 100 %) zu einem Vorgemisch mischt und gegebenenfalls
(ii) danach in die Vormischung 0,1 bis 1,1 Gew.-% Vernetzer (bezogen auf (a) und (b) als 100 %) einarbeitet,
(iii) das anfallende Gemisch unter Druck einer Heißvulkanisation unterwirft,
(iv) das resultierende Vulkanisat einer Wärmebehandlung unterwirft und
(v) das wärmebehandelte Vulkanisat zu einem erfindungsgemäßen Werkstoff verarbeitet.

Stufe (ii) kann man mit Hilfe eines Walzwerks durchführen.

Werden Farbpigmente vorgesehen, so können sie bei Stufe (ii) eingearbeitet werden.

Zur Heißvulkanisation der Stufe (iii) kann man das bei Stufe (ii) anfallende Gemisch einem Extrusions-, einem Spritzgieß- oder einem Preßvorgang unterwerfen. Zur Heißvulkanisation kann man eine Temperatur im Bereich von 150 bis 180 °C und/oder einen Druck im Bereich von 50 bis 140 Tonnen pro 225 cm², insbesondere etwa 100 Tonnen pro 225 cm² anwenden.

### Beispiel

Zur Herstellung von Kofferdam wählte man folgende Komponenten:
8,0 kg Silikonkautschuk
2,0 kg hochaktive pyrogene Kieselsäure
0,4 kg Silikonöl

Die genannten Komponenten wurden mit Hilfe eines Walzwerks zu einem Vorgemisch vermischt. Danach gab man zur Vormischung 60 g eines in der Gummiindustrie üblichen Peroxids als Vernetzer sowie 10 g eines anorganischen Grün-Pigments, wobei diese zusätzlichen Komponenten mit Hilfe des Walzwerks eingearbeitet wurden.

Das anfallende Gemisch wurde unter Druck in Formen einer Heißvulkanisation unterworfen, wobei Platten von 150 x 150 x 0,5 mm anfielen. Diese Platten wurden einer Wärmebehandlung ausgesetzt, um unumgesetzten Vernetzer zu zersetzen.

Die hergestellten Kofferdam-Platten waren transparent.

## Patentansprüche

1. Stoffgemisch für einen Kofferdam, **gekennzeichnet durch**
(a) 56 bis 96 Gew.-% Silikonkautschuk (Polymer),
(b) 44 bis 4 Gew.-% Kieselsäure (Füllstoff),
(c) 1 bis 7 Gew.-% übliches Silikonkautschuk-Verarbeitungshilfsmittel (bezogen auf (a) und (b) als 100 %) und
(d) 0,1 bis 1,1 Gew.-% eines Radikale bildenden Vernetzers (bezogen auf (a) und (b) als 100 %), wobei der Vernetzer im Gemisch mit den Komponenten (a) bis (c) oder getrennt davon vorliegen kann.

2. Stoffgemisch nach Anspruch 1, **gekennzeichnet durch** 8 bis 28 und insbesondere 20 Gew.-% Kieselsäure.

3. Stoffgemisch nach Anspruch 1 oder 2, **gekennzeichnet durch** ein Öl, insbesondere ein Silikonöl, als Silikonkautschuk-Verarbeitungshilfsmittel.

4. Stoffgemisch nach einem der vorhergehenden Ansprüche, **gekennzeichnet durch** ein Peroxid als Vernetzer, insbesondere einen in der Gummiindustrie üblichen Vernetzer.

5. Stoffgemisch nach einem der vorhergehenden Ansprüche, **gekennzeichnet durch** einen zusätzlichen Gehalt an Farbpigment.

6. Stoffgemisch nach einem der vorhergehenden Ansprüche, **gekennzeichnet durch**
(a) etwa 80 Gew.-% Silikonkautschuk,
(b) etwa 20 Gew.-% Kieselsäure,
(c) etwa 4 Gew.-% Silikonkautschuk-Verarbeitungshilfsmittel (bezogen auf (a) und (b) als 100 %) und
(d) etwa 0,6 Gew.-% Vernetzer (bezogen auf (a) und (b) als 100 %).

7. Werkstoff für Kofferdam, erhalten durch Vulkanisation aus einem Stoffgemisch gemäß einem der vorhergehenden Ansprüche.

8. Werkstoff für Kofferdam, erhalten durch Vulkanisation aus einem Stoffgemisch gemäß einem der Ansprüche 1 bis 6 mit den Merkmalen (a) bis (c) und mit einem Metall, insbesondere Platin, als Vernetzer.

9. Werkstoff nach Anspruch 7 oder 8, **dadurch gekennzeichnet, daß** er transparent ist.

10. Werkstoff nach einem der Ansprüche 7 bis 9, **dadurch gekennzeichnet, daß** er als Flachmaterial vorgesehen ist.

11. Werkstoff nach einem der Ansprüche 7 bis 10, **dadurch gekennzeichnet, daß** er in Form runder, rechteckiger oder quadratischer Platten vorliegt, insbesondere mit den Ausmaßen von etwa 150 x etwa 150 mm.

12. Werkstoff nach einem der Ansprüche 7 bis 11, **dadurch gekennzeichnet, daß** er eine Stärke von 0,1 bis 0,8 mm besitzt.

13. Verwendung des Werkstoffs gemäß einem der Ansprüche 7 bis 12 für Kofferdam.

14. Verfahren zur Herstellung eines Stoffgemischs für einen Kofferdam-Werkstoff, **dadurch gekennzeichnet, daß** man
(i)
(a) 56 bis 96 Gew.-% Silikonkautschuk (Polymer),
(b) 44 bis 4 Gew.-% Kieselsäure (Füllstoff) und
1 bis 7 Gew.-% übliches Silikonkautschuk-Verarbeitungshilfsmittel (bezogen auf (a) und (b) als 100 %) zu einem Vorgemisch mischt und gegebenenfalls
(ii) danach in die Vormischung 0,1 bis 1,1 Gew.-% Vernetzer (bezogen auf (a) und (b) als 100 %) einarbeitet,
(iii) das anfallende Gemisch unter Druck einer Heißvulkanisation unterwirft,
(iv) das resultierende Vulkanisat einer Wärmebehandlung unterwirft und
(v) das wärmebehandelte Vulkanisat zu einem Werkstoff gemäß einem der Ansprüche 7 bis 12 verarbeitet.

15. Verfahren nach Anspruch 14, **dadurch gekennzeichnet, daß** man die Stufe (ii) mit Hilfe eines Walzwerks durchführt.

16. Verfahren nach Anspruch 14 oder 15, **dadurch gekennzeichnet, daß** man bei der Stufe (ii) Farbpigmente einarbeitet.

17. Verfahren nach einem der Ansprüche 14 bis 16, **dadurch gekennzeichnet, daß** man das bei der Stufe (ii) anfallende Gemisch zur Heißvulkanisation einem Extrusions-, Spritzgieß- oder Preßvorgang unterwirft.

18. Verfahren nach einem der Ansprüche 14 bis 17, **dadurch gekennzeichnet, daß** man die Heißvulkanisation der Stufe (iii) bei einer Temperatur im Bereich von 150 bis 180 °C und/oder einem Druck im Bereich von 50 bis 140 Tonnen/225 cm² und insbesondere etwa 100 Tonnen/225cm² durchführt.

## Claims

1. Substance mixture for a cofferdam, **characterised by**
(a) from 56 to 96 % by weight silicone rubber (polymer),
(b) from 44 to 4 % by weight silicic acid (filler),
(c) from 1 to 7 % by weight customary silicone rubber processing auxiliary (based on (a) and (b) as 100 %) and
(d) from 0.1 to 1.1 % by weight free-radical-forming cross-linking agent (based on (a) and (b) as 100 %), it being possible for the cross-linking agent to be in admixture with components (a) to (c) or to be separate therefrom.

2. Substance mixture according to claim 1, **characterised by** from 8 to 28 %, and especially 20 %, by weight silicic acid.

3. Substance mixture according to claim 1 or 2, **characterised by** an oil, especially a silicone oil, as silicone rubber processing auxiliary.

4. Substance mixture according to any one of the preceding claims, **characterised by** a peroxide as cross-linking agent, especially a cross-linking agent customary in the rubber industry.

5. Substance mixture according to any one of the preceding claims, **characterised by** an additional content of colour pigment.

6. Substance mixture according to any one of the preceding claims, **characterised by**
(a) approximately 80 % by weight silicone rubber,
(b) approximately 20 % by weight silicic acid,
(c) approximately 4 % by weight silicone rubber processing auxiliary (based on (a) and (b) as 100 %) and
(d) approximately 0.6 % by weight cross-linking agent (based on (a) and (b) as 100%).

7. Material for cofferdam, obtained by vulcanisation from a substance mixture according to any one of the preceding claims.

8. Material for cofferdam, obtained by vulcanisation from a substance mixture according to any one of claims 1 to 6 having features (a) to (c) and having a metal, especially platinum, as cross-linking agent.

9. Material according to claim 7 or 8, **characterised in that** it is transparent.

10. Material according to any one of claims 7 to 9, **characterised in that** it is provided in the form of a flat material.

11. Material according to any one of claims 7 to 10, **characterised in that** it is in the form of round, rectangular or square sheets, especially with dimensions of approximately 150 mm x approximately 150 mm.

12. Material according to any one of claims 7 to 11, **characterised in that** it has a thickness of from 0.1 to 0.8 mm.

13. Use of the material according to any one of claims 7 to 12 for cofferdam.

14. Process for the preparation of a substance mixture for a cofferdam material, **characterised in that**
(i)
(a) from 56 to 96 % by weight silicone rubber (polymer),
(b) from 44 to 4 % by weight silicic acid (filler) and
from 1 to 7 % by weight customary silicone rubber processing auxiliary (based on (a) and (b) as 100 %)
are mixed to form a premix and optionally
(ii) from 0.1 to 1.1 % by weight cross-linking agent (based on (a) and (b) as 100 %) is then incorporated into the premix,
(iii) the resulting mixture is subjected to hot vulcanisation under pressure,
(iv) the resulting vulcanisate is subjected to a heat treatment, and
(v) the heat-treated vulcanisate is processed to form a material according to any one of claims 7 to 12.

15. Process according to claim 14, **characterised in that** step (ii) is carried out with the aid of a roll mill.

16. Process according to claim 14 or 15, **characterised in that** colour pigments are incorporated in step (ii).

17. Process according to any one of claims 14 to 16, **characterised in that** the mixture obtained in step (ii) is subjected to an extrusion, injection-moulding or compression operation for the purpose of hot vulcanisation.

18. Process according to any one of claims 14 to 17, **characterised in that** the hot vulcanisation of step (iii) is carried out at a temperature in the range of from 150 to 180°C and/or at a pressure in the range of from 50 to 140 tonnes/225 cm² and especially approximately 100 tonnes/225 cm².

## Revendications

1. Mélange de matériaux pour une digue en caoutchouc, **caractérisé par** :
(a) 56 à 96 % en poids de caoutchouc au silicone (polymère),
(b) 44 à 4 % en poids d'acide silicique (matériau de remplissage),
(c) 1 à 7 % en poids d'agent auxiliaire de traitement pour caoutchouc au silicone habituel (par référence à (a) et (b) représentant 100 %), et
(d) 0,1 à 1,1 % en poids d'un agent de réticulation formant un radical (par référence à (a) et (b) représentant 100 %), ledit agent de réticulation pouvant être présent dans le mélange avec les composants (a) à (c), ou séparé de ceux-ci.

2. Mélange de matériaux selon la revendication 1, **caractérisé par** 8 à 28 %, et en particulier par 20 % en poids d'acide silicique.

3. Mélange de matériaux selon l'une ou l'autre des revendications 1 et 2, **caractérisé par** une huile, en particulier une huile au silicone à titre d'agent auxiliaire de traitement pour caoutchouc au silicone.

4. Mélange de matériaux selon l'une des revendications précédentes, **caractérisé par** un peroxyde à titre d'agent de réticulation, en particulier par un agent de réticulation habituel dans l'industrie du caoutchouc.

5. Mélange de matériaux selon l'une des revendications précédentes, **caractérisé par** une teneur additionnelle de pigment coloré.

6. Mélange de matériaux selon l'une des revendications précédentes, **caractérisé par** :
(a) environ 80 % en poids de caoutchouc au silicone,
(b) environ 20 % en poids d'acide silicique,
(c) environ 4 % en poids d'agent auxiliaire de traitement pour caoutchouc au silicone (par référence à (a) et (b) représentant 100 %), et
(d) environ 0,6 % en poids d'agent de réticulation (par référence à (a) et (b) représentant 100 %).

7. Matériau pour digue en caoutchouc, obtenu par vulcanisation d'un mélange de matériaux selon l'une des revendications précédentes.

8. Matériau pour digue en caoutchouc, obtenu par vulcanisation d'un mélange de matériaux selon l'une des revendications 1 à 6, présentant les caractéristiques (a) à (c), et comprenant un métal, en particulier du platine, à titre d'agent de réticulation.

9. Matériau selon l'une ou l'autre des revendications 7 et 8, **caractérisé en ce qu'**il est transparent.

10. Matériau selon l'une des revendications 7 à 9, **caractérisé en ce qu'**il est prévu à titre de matériau plat.

11. Matériau selon l'une des revendications 7 à 10, **caractérisé en ce qu'**il est présent sous la forme de plaques rondes, rectangulaires ou carrées, en particulier avec des dimensions d'environ 150 x 150 mm.

12. Matériau selon l'une des revendications 7 à 11, **caractérisé en ce qu'**il possède une épaisseur de 0,1 à 0,8 mm.

13. Application du matériau selon l'une des revendications 7 à 12 pour une digue en caoutchouc.

14. Procédé pour la préparation d'un mélange de matériaux pour un matériau de digue en caoutchouc,
**caractérisé par** les opérations suivantes :
(i) on mélange :
(a) 56 à 96 % en poids de caoutchouc au silicone (polymère),
(b) 44 à 4 % en poids d'acide silicique (matériau de remplissage), et
1 à 7 % en poids d'agent auxiliaire de traitement de caoutchouc au silicone habituel (par référence à (a) et (b) représentant 100 %) en un mélange précurseur, et en option
(ii) on intègre ensuite dans le mélange précurseur 0,1 à 1,1 % en poids d'agent de réticulation (par référence à (a) et (b) représentant 100 %),
(iii) on soumet le mélange résultant sous pression à une vulcanisation à chaud,
(iv) on soumet le produit vulcanisé résultant à un traitement thermique, et
(v) on transforme le produit vulcanisé traité à chaud en un matériau selon l'une des revendications 7 à 12.

15. Procédé selon la revendication 14, **caractérisé en ce que** l'on exécute l'opération (ii) à l'aide d'un laminoir.

16. Procédé selon l'une ou l'autre des revendications 14 et 15, **caractérisé en ce que** l'on intègre des pigments colorés lors de l'opération (ii).

17. Procédé selon l'une des revendications 14 à 16, **caractérisé en ce que** l'on soumet le mélange résultant de l'opération (ii) à une opération d'extrusion, de coulée-injection, ou de pressage, pour sa vulcanisation à chaud.

18. Procédé selon l'une des revendications 14 à 17, **caractérisé en ce que** l'on exécute la vulcanisation à chaud de l'opération (iii) à une température dans la plage de 150 à 180°C et/ou sous une pression dans la plage de 50 à 140 tonnes/225 cm², et en particulier environ 100 tonnes/225 cm².
